# EUROPÄISCHE PATENTSCHRIFT

(11) **EP 2 120 690 B1**
(45) Veröffentlichungstag und Bekanntmachung des Hinweises auf die Patenterteilung: **21.09.2011**
(21) Anmeldenummer: 08700987.4
(22) Anmeldetag: 05.01.2008
(51) Int. Cl.: A61B 5/03

(54) **MESSEINRICHTUNG FÜR PHYSIOLOGISCHE PARAMETER**
MEASUREMENT DEVICE FOR PHYSIOLOGICAL PARAMETERS
INSTRUMENT DE MESURE DE PARAMETRES PHYSIOLOGIQUES

(30) Priorität: 22.02.2007 DE 102007008642
(43) Veröffentlichungstag der Anmeldung: 25.11.2009
(73) Patentinhaber: Aesculap AG, 78532 Tuttlingen/Donau (DE)
(72) Erfinder: LUTZE, Theodor, 78582 Balgheim (DE); SCHAUER, Dirk, 10318 Berlin (DE); MIETHKE, Christoph, 14469 Potsdam (DE)
(74) Vertreter: Hoeger, Stellrecht & Partner Patentanwälte
(86) Internationale Anmeldenummer: PCT/EP2008/000039
(87) Internationale Veröffentlichungsnummer: WO 2008/101563

(56) Entgegenhaltungen:
- WO-A-2006/117123
- US-A1- 2005 187 488

## Beschreibung

Die Erfindung betrifft eine Messeinrichtung für physiologische Parameter mit einer Hirnflüssigkeit aufnehmenden, implantierbaren Flüssigkeitskammer, mit einer mit der Hirnflüssigkeit in der Flüssigkeitskammer über eine Membran in Verbindung stehenden Sensoreinrichtung, die einen Sensor für einen oder mehrere physiologische Parameter, elektronische Bauelemente und eine Telemetrieeinrichtung zur drahtlosen Übertragung von Signalen des Sensors umfasst.

Eine solche Messeinrichtung ist beispielsweise beschrieben in der WO 2006/117123 A1. Derartige Messeinrichtungen werden üblicherweise außerhalb des Schädelknochens und unterhalb der Kopfhaut implantiert, teils unmittelbar über einem Bohrloch in der Schädeldecke, teils neben einem derartigen Bohrloch, durch welches Hirnflüssigkeit aus dem Schädelinneren in die Flüssigkeitskammer eintreten kann. Bei der vorbekannten Messeinrichtung ist die Flüssigkeitskammer als Doppelkammer ausgebildet, die durch eine Zwischenwand in zwei Abteile unterteilt wird, eines der Abteile nimmt die Sensoreinrichtung auf, das andere Abteil wird von der Hirnflüssigkeit durchströmt. In der Trennwand zwischen den Abteilen ist eine Membran angeordnet, durch die der Druck der Hirnflüssigkeit auf einen Drucksensor der Sensoreinrichtung übertragen wird.

Eine solche Anordnung muss insgesamt vorgefertigt werden und muss hermetisch abgeschlossen sein, so dass im Falle einer Fehlfunktion nur ein vollständiger Austausch möglich ist. Außerdem ist die Herstellung aufwendig. Es ist Aufgabe der Erfindung, eine gattungsgemäße Messeinrichtung so auszubilden, dass die Herstellung vereinfacht wird und dass gegebenenfalls Reparaturarbeiten vorgenommen werden können, ohne die gesamte Messeinrichtung auswechseln zu müssen.

Diese Aufgabe wird bei einer Messeinrichtung der eingangs beschriebenen Art erfindungsgemäß dadurch gelöst, dass die Flüssigkeitskammer mindestens zwei Gehäuseteile aufweist, die unter Ausbildung eines geschlossenen Innenraums dichtend zusammenfügbar sind und die im nicht zusammengefügten Zustand einen Zugang zu dem Innenraum ermöglichen, und dass die Sensoreinrichtung in einer allseits geschlossenen Messkammer angeordnet ist, die als selbständig handhabbares Bauteil ausgebildet und in einer definierten Position im Innenraum der Flüssigkeitskammer einsetzbar ist.

Durch die Ausbildung der Messkammer als selbständig handhabbares Bauteil kann diese in den geöffneten Innenraum der Flüssigkeitskammer eingesetzt werden und wird dann in diesem in einer definierten Position gehalten. Durch Verbinden der mindestens zwei Gehäuseteile der Flüssigkeitskammer wird die Flüssigkeitskammer dann nach außen dicht abgeschlossen, kann aber auch wieder geöffnet werden, falls dies notwendig ist, so dass die Messkammer herausgenommen und gegebenenfalls ausgewechselt werden kann, ohne dass dazu ein Auswechseln der Flüssigkeitskammer notwendig ist. Diese kann auch bei einer Fehlfunktion der Sensoreinrichtung in ihrer implantierten Stellung verbleiben und nach Einsetzen einer neuen Messkammer wieder weiterverwendet werden.

Der Operateur hat so auch die Möglichkeit, die Messkammer erst während der Operation einzusetzen, wenn die schwierige Implantation der Flüssigkeitskammer abgeschlossen ist. Dies erleichtert die Operation und schont die empfindliche Sensoreinrichtung während der Operation.

Günstig ist es, wenn der Innenraum über mindestens einen Zufluss mit einer Sonde in Verbindung steht, über die Hirnflüssigkeit von einer Messstelle des Hirns in den Innenraum einströmen kann. Außerdem kann vorgesehen sein, dass der Innenraum mindestens einen Abfluss für Hirnflüssigkeit aufweist. Die Flüssigkeitskammer ist dann Teil eines Shunt-Systems, mit dem in an sich bekannter Weise Hirnflüssigkeit aus dem Hirn abgeführt wird, beispielsweise über eine Abflussleitung in den Bauchraum.

Bei einer bevorzugten Ausführungsform ist vorgesehen, dass die Flüssigkeitskammer ein erstes Gehäuseteil mit einem Boden und daran anschließenden Seitenwänden und ein zweites Gehäuseteil aufweist, welches als dichtend auf das erste Gehäuseteil aufsetzbarer Deckel ausgebildet ist.

Besonders vorteilhaft ist es, wenn ein Bereich der Wand der Flüssigkeitskammer flexibel ausgebildet ist, während die übrigen Bereiche der Wand starr ausgebildet sind. Auf diese Weise ist es möglich, durch Eindrücken des flexiblen Wandbereiches einen Druckimpuls auf die Hirnflüssigkeit in der Flüssigkeitskammer zu übertragen. Dieser Druckimpuls kann als Pumpimpuls wirken und somit beispielsweise bei Verunreinigungen und Verstopfungen der Strömungswege zu einer Reinigung führen, der Druckimpuls kann aber auch verwendet werden, um beispielsweise Kontrollsignale des Sensors zu erzeugen und um dadurch dessen Funktionsfähigkeit zu überprüfen.

Insbesondere kann der flexible Wandbereich durch eine flexible Membran gebildet werden, beispielsweise eine flexible Membran aus Silikon.

Eine solche Ausbildung hat weiterhin den Vorteil, dass durch eine solche flexible Membran aus Silikon oder einem ähnlichen Material eine Kanüle in den Innenraum der Flüssigkeitskammer eingeführt werden kann, mit der Proben der Flüssigkeit entnommen werden können. Auf diese Weise ist auch bei implantierter Messeinrichtung möglich, durch die Kopfhaut hindurch und durch die flexible Membran hindurch Zugang zu der Hirnflüssigkeit zu erlangen.

Gemäß einer bevorzugten Ausführungsform kann die flexible Membran in einen Dichtring eingesetzt sein, der zusammen mit der Membran ein Gehäuseteil ausbildet, welches mit einem zweiten Gehäuseteil zur Bildung der Flüssigkeitskammer zusammenfügbar ist. Dieser Dichtring kann beispielsweise in das zweite Gehäuseteil dichtend eingeschraubt sein.

Insbesondere bei einer Ausgestaltung mit einem flexiblen Wandbereich der Flüssigkeitskammer ist es vorteilhaft, wenn die Messkammer derart in die Flüssigkeitskammer eingesetzt ist, dass sie allseits von Hirnflüssigkeit umgeben ist. Dadurch wird die Pumpmöglichkeit mit Hilfe des flexiblen Wandbereichs verbessert, da auch der unmittelbar an den flexiblen Wandbereich angrenzende Teil des Innenraums von der Hirnflüssigkeit durchströmt wird.

Die Messkammer besteht vorzugsweise aus Metall, beispielsweise aus Titan oder einer Titanlegierung, und es ist günstig, wenn die Messkammer starre Wände aufweist. Dabei können die Wände trotzdem relativ dünn sein, beispielsweise liegt die Wandstärke in der Größenordnung von einigen zehntel Millimetern.

Die Flüssigkeitskammer kann ebenfalls aus einem körperverträglichen Metall bestehen, beispielsweise aus Titan oder einer Titanlegierung, es ist aber auch möglich, die Flüssigkeitskammer aus einem sterilisierbaren Kunststoff herzustellen, beispielsweise aus Polyetheretherketon. Die Verwendung von Kunststoff hat den Vorteil, dass die Abschirmwirkung für die Telemetrieeinrichtung im Inneren der Messkammer entfällt, die bei einer metallischen Ausbildung des Flüssigkeitsraumes gegeben ist und die die Übertragung der Signale erschwert.

Die nachfolgende Beschreibung bevorzugter Ausführungsformen der Erfindung dient im Zusammenhang mit der Zeichnung der näheren Erläuterung. Es zeigen:

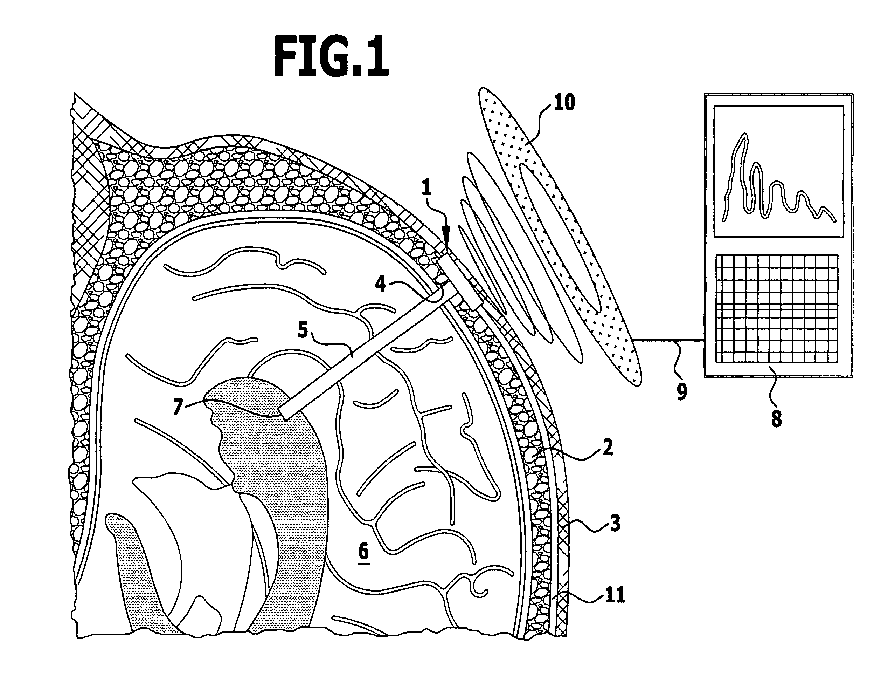

- Figur 1:: eine schematische Schnittansicht durch den Schädel eines Patienten mit einer implantierten Messeinrichtung und einer ex ternen Auswerteeinrichtung;
- Figur 2:: eine schematische Seitensicht einer Messeinrichtung mit einer in das Gehirn einsetzbaren Sonde und mit einer Drainage-Leitung für die Hirnflüssigkeit bei einer über einem Bohrloch in der Schädeldecke einsetzbaren Messeinrichtung;
- Figur 3:: eine Ansicht ähnlich Figur 2 bei einer neben einem Bohrloch in der Schädeldecke implantierbaren Messeinrichtung;
- Figur 4:: eine Schnittansicht einer geschlossenen Flüssigkeitskammer ohne eingesetzte Messkammer;
- Figur 5:: eine Ansicht ähnlich Figur 4 mit eingesetzter Messkammer;

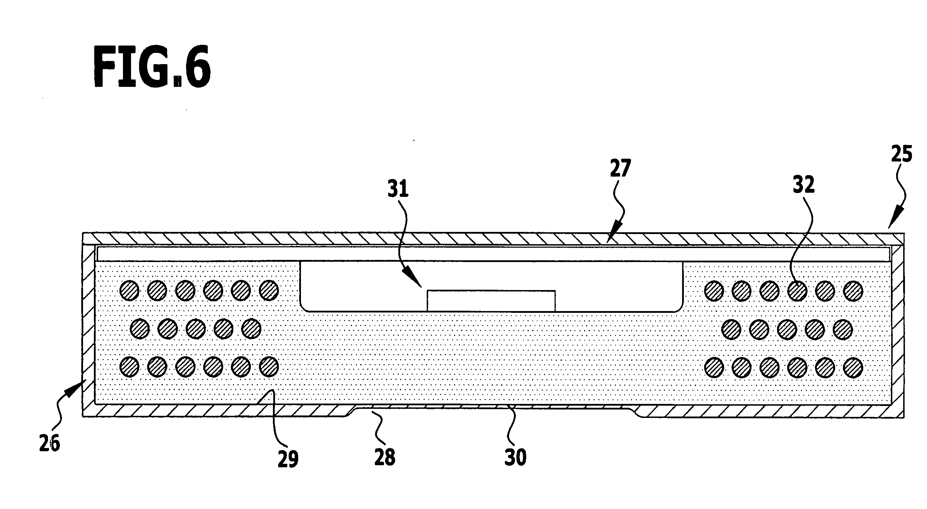

- Figur 6:: eine Längsschnittansicht durch eine Messkammer;
- Figur 7:: eine Schnittansicht einer leeren und unverschlossenen Mess- kammer;

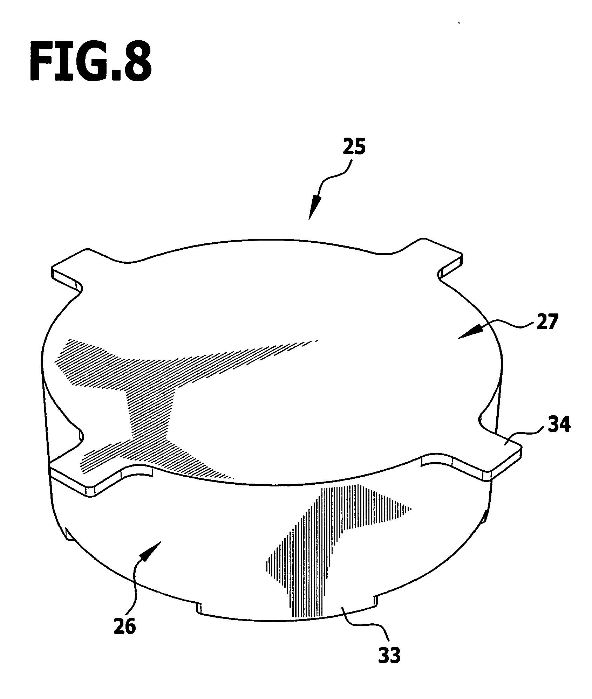

- Figur 8:: eine perspektivische Ansicht einer verschlossenen Messkammer;
- Figur 9:: eine Längsschnittansicht ähnlich Figur 5 einer von Hirnflüssigkeit durchströmten Messeinrichtung;
- Figur 10:: eine Ansicht ähnlich Figur 9 mit der Darstellung der Strömung der Hirnflüssigkeit beim Eindrücken eines flexiblen Wand- bereiches und
- Figur 11:: eine Explosionsansicht eines weiteren bevorzugten Ausführungs- beispiels einer Messeinrichtung.

Die in der Zeichnung dargestellte Messeinrichtung 1 wird üblicherweise außerhalb des Schädelknochens 2 und unterhalb der Kopfhaut 3 über einem Bohrloch 4 oder neben einem solchen Bohrloch implantiert. Durch das Bohrloch 4 hindurch reicht eine Sonde 5 oder ein Katheter in das Innere des Gehirns 6 und endet an einer Einlassstelle 7, an der Hirnflüssigkeit aus dem Gehirn 6 in die Sonde 5 eintreten kann. Diese Hirnflüssigkeit wird durch die Sonde 5 der Messeinrichtung 1 zugeführt, so dass in der Messeinrichtung 1 physiologische Werte der Hirnflüssigkeit gemessen werden können, beispielsweise der Druck der Hirnflüssigkeit und/oder die Temperatur.

Die auf diese Weise in der Messeinrichtung 1 aufgenommenen Messwerte der physiologischen Parameter werden drahtlos an eine externe Auswerteeinrichtung übertragen, die über eine Messleitung 9 mit einer Spule 10 verbunden ist. Diese Spule 10 kann in der Nähe der Messeinrichtung 1 so angeordnet werden, dass sie drahtlos Signale mit einer entsprechenden Spule der Messeinrichtung austauschen kann. Über diese Spulen kann auch die Messeinrichtung 1 mit externer Energie versorgt werden.

Der Messeinrichtung 1 zugeführte Hirnflüssigkeit kann über eine Drainageleitung 11 abgeführt werden, beispielsweise endet eine solche Drainageleitung in der Bauchhöhle des Patienten. Die Sonde 5, die Messeinrichtung 1 und die Drainageleitung 11 bilden somit ein Shunt-System, mit dem Hirnflüssigkeit aus dem Schädel entfernt werden kann.

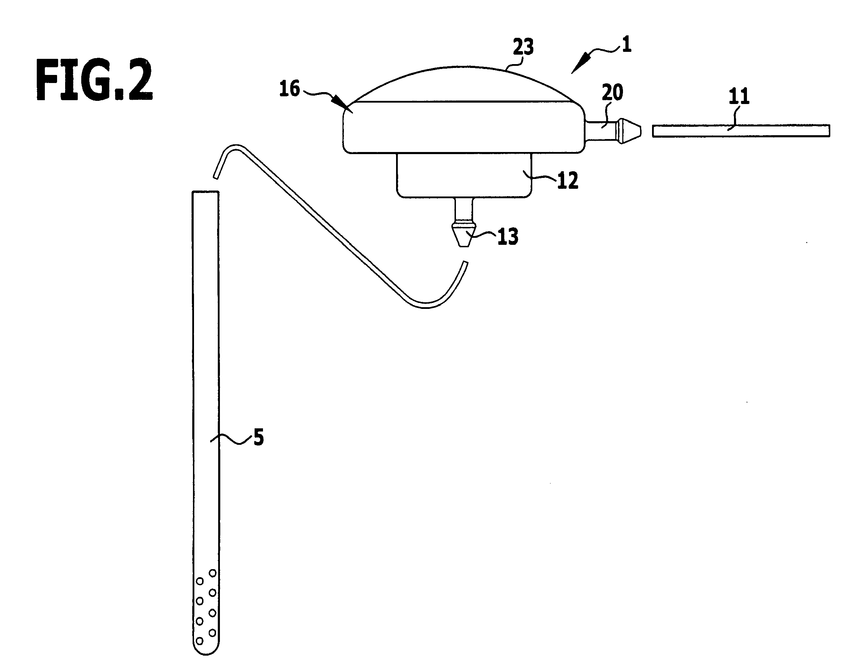

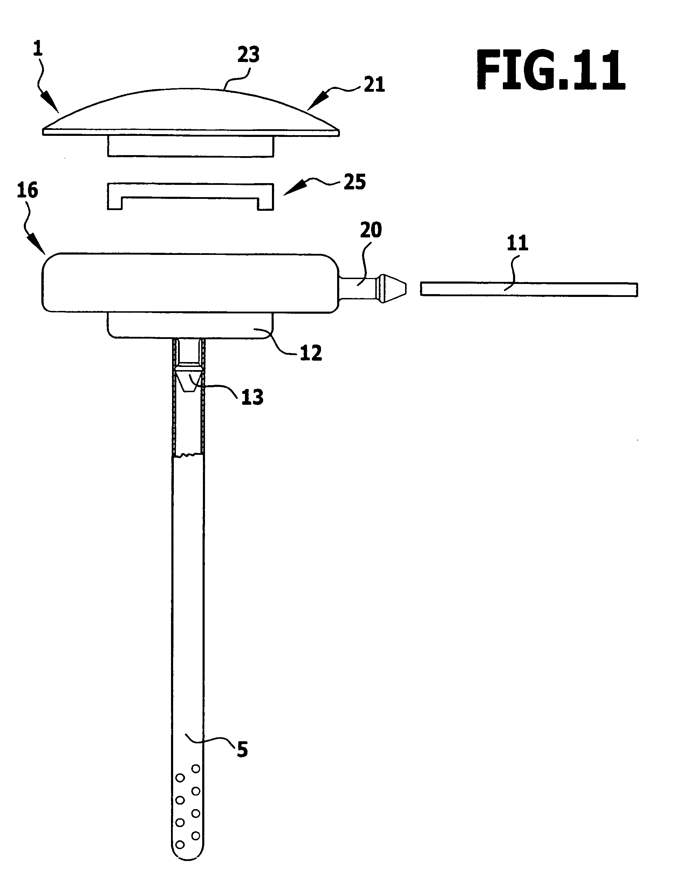

Wenn die Messeinrichtung 1 unmittelbar über dem Bohrloch 4 platziert wird, dann ist es günstig, wenn die Messeinrichtung 1 an ihrer Unterseite einen in das Bohrloch 4 eintauchenden Vorsprung 12 aufweist sowie einen sich daran anschließenden Anschluss 13, der beispielsweise über eine flexible Leitung mit der Sonde 5 verbunden sein kann (Figur 2) oder auf den die Sonde 5 unmittelbar aufgeschoben ist (Figur 11).

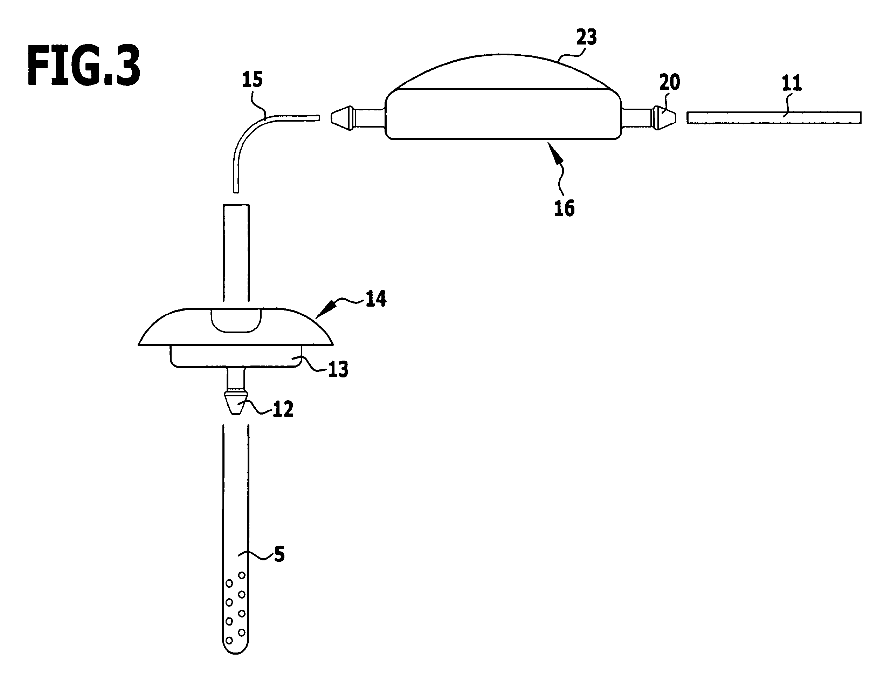

Wenn dagegen die Messeinrichtung 1 neben einem Bohrloch 4 angeordnet wird, ist es vorteilhaft, wenn eine Bohrlochabdeckung 14 vorgesehen ist, die an ihrer Unterseite ebenfalls einen Vorsprung 12 und einen Anschluss 13 trägt und die eine Umlenkung der Hirnflüssigkeit parallel zum Schädelknochen vornimmt, so dass die Hirnflüssigkeit über eine weitere Leitung 15 seitlich in die Messeinrichtung 1 eintritt (Figur 3).

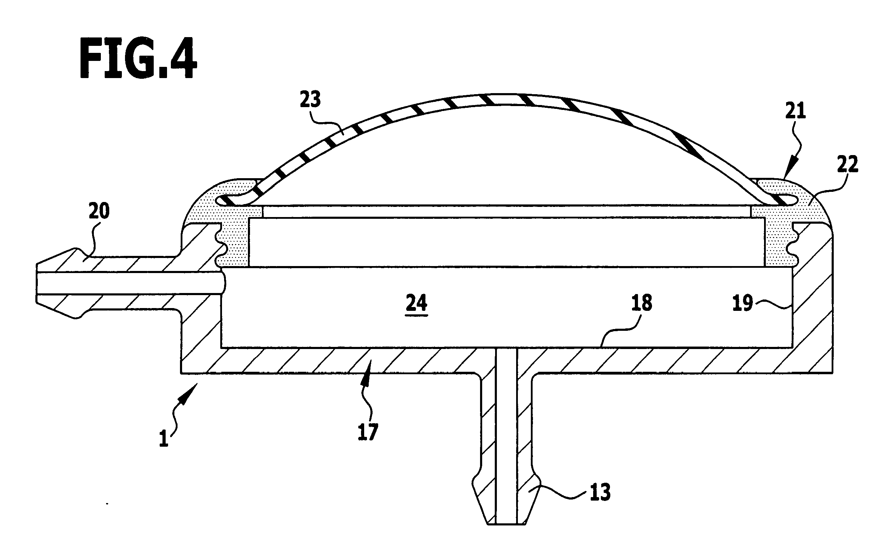

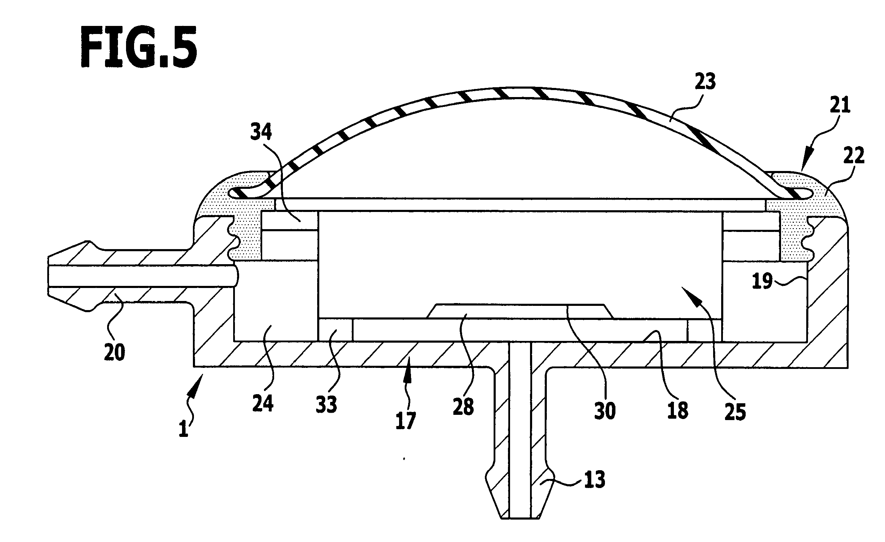

Die Messeinrichtung 1 umfasst eine Flüssigkeitskammer 16 mit einem dosenförmigen Unterteil 17 mit einem ebenen Boden 18 und außenseitig daran anschließenden senkrechten Wänden 19, vorzugsweise hat dieses dosenförmige Unterteil 17 einen kreisförmigen Querschnitt. Bei dem in den Figuren 4 und 5 dargestellten Ausführungsbeispiel trägt der Boden 18 einen nach unten gerichteten Anschluss 13 für den Zufluss der Hirnflüssigkeit und einen horizontal aus der Wand 19 austretenden Auslass 20 für den Abfluss der Hirnflüssigkeit.

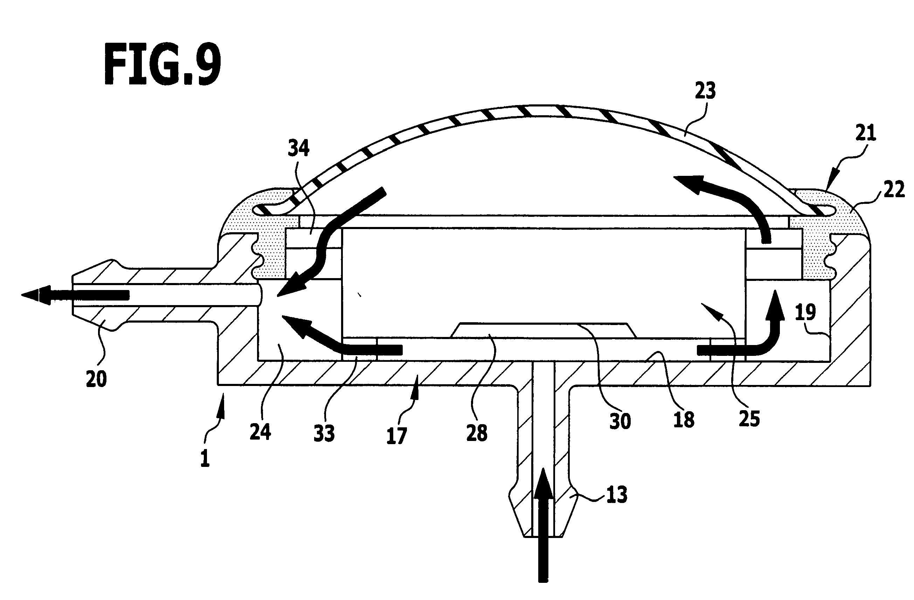

Das dosenförmige Unterteil 17 ist an der Oberseite offen und kann dort durch ein deckelförmiges Gehäuseteil 21 dicht verschlossen werden. Dieses deckelförmige Gehäuseteil 21 umfasst einen Ring 22, der als Dichtring ausgebildet ist und dichtend an die Wand 19 des Unterteils 17 anschließbar ist, beispielsweise durch Einschrauben oder durch Einkleben. In den Ring 22 ist abgedichtet eine haubenförmige flexible Membran 23 eingesetzt, die vorzugsweise aus Silikon besteht, während der Ring 22 beispielsweise aus Polyetheretherketon bestehen kann. Im zusammengefügten Zustand bilden das bodenförmige Unterteil 17 und das deckelförmige Gehäuseteil 21 eine gehäuseartige Flüssigkeitskammer aus mit einem Innenraum 24, der bis auf den Anschluss 13 und den Auslass 20 geschlossen ist. Dieser Innenraum 24 wird im implantierten Zustand von der Hirnflüssigkeit durchströmt und ist vollständig von ihr ausgefüllt. Dabei wird die flexible Membran 23 in der in Figur 9 dargestellten Weise nach außen gewölbt.

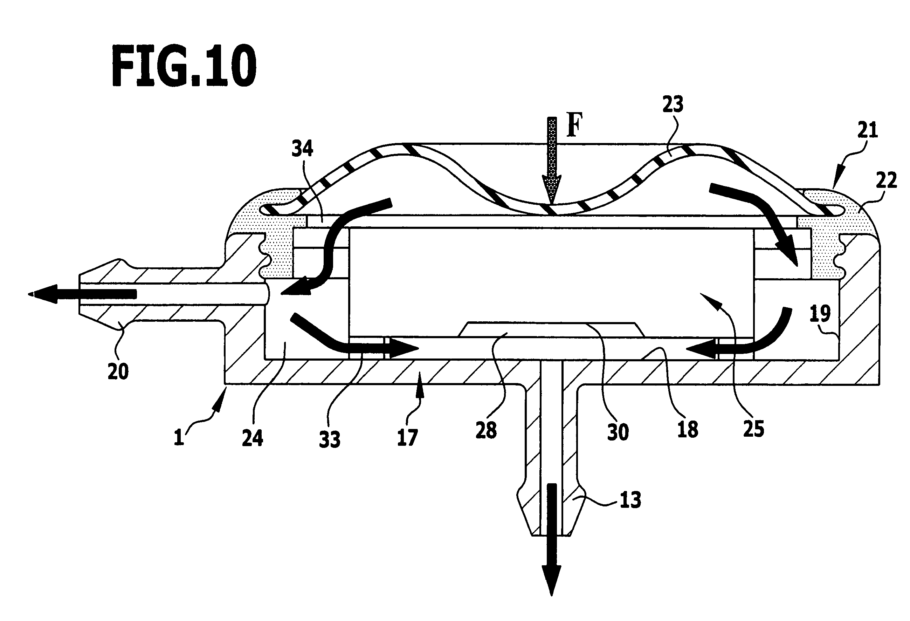

Durch Druck auf die Membran 23 kann diese flexible Membran nach innen gedrückt werden, wie dies in Figur 10 dargestellt ist. Dadurch wird die Hirnflüssigkeit aus dem Innenraum 24 sowohl über den Auslass 20 als auch über den Anschluss 13 aus der Flüssigkeitskammer 16 heraus gedrängt, d.h. es erfolgt eine Rückspülung der Sonde 5 und eine verstärkte Durchspülung der Drainageleitung 11. Dieser Spüleffekt kann zur Reinigung der entsprechenden Leitungen eingesetzt werden, es ist aber auch möglich, durch diesen Druck auf die flexible Membran 23 einen Druckimpuls auf die Hirnflüssigkeit zu übertragen, der dann von der Messeinrichtung 1 registriert werden kann. Dadurch lässt sich die Messeinrichtung in ihrer Funktion kontrollieren.

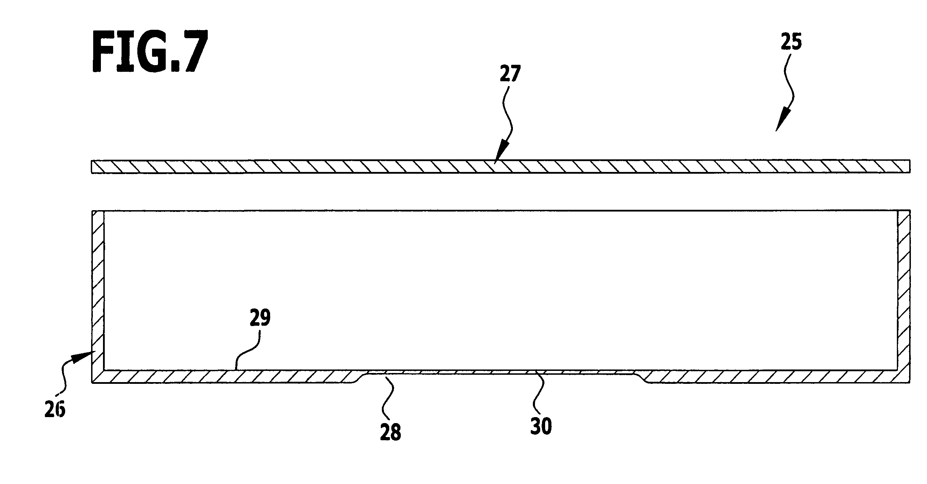

In den Innenraum 24 ist eine vollständig abgeschlossene Messkammer 25 eingesetzt, die ein ebenfalls dosenförmiges und im Querschnitt kreisförmiges Unterteil 26 und einen auf dieses Unterteil 26 abgedichtet aufgesetzten Deckel 27 umfasst (Figur 7). Diese Messkammer besteht vorzugsweise aus Metall, insbesondere aus Titan oder einer Titanlegierung, die Wände sind starr ausgebildet mit Ausnahme eines zentralen Bereiches 28 am Boden 29 des Unterteils 26. In diesem Bereich ist entweder eine flexible Membran 30 eingesetzt oder aber der Boden 29 durch Materialabtragung so dünn ausgebildet, dass er eine flexible Membran 30 bildet.

Im Inneren der Messkammer 25 sind ein Sensorchip 31 sowie eine diesen umgebende Spule 32 aufgenommen. Der Sensorchip 31 trägt mindestens einen Sensor, beispielsweise einen Drucksensor und/oder einen Temperatursensor, außerdem elektronische Bauelemente zur Verarbeitung und Digitalisierung der von den Sensoren erzeugten elektrischen Signale sowie zur Energieversorgung, weiterhin elektronische Bauelemente einer Telemetrieschaltung, die die von den Sensoren erzeugten Signale über die Spule 32 nach außen sendet, diese Signale können dann von der Spule 10 der externen Auswerteeinrichtung 8 empfangen werden.

Der Innenraum der Messkammer 25 ist mit einem druckübertragenden Medium gefüllt, beispielsweise mit Luft, mit einem Öl oder mit einem Gel, es ist auch möglich, dass zwischen der flexiblen Membran 30 und den Sensoren auf dem Sensorchip 31 mechanische Übertragungsglieder vorgesehen sind, beispielsweise Kolben oder Hebel, wesentlich ist lediglich, dass der Druck der Hirnflüssigkeit über die flexible Membran 30 die Sensoren auf dem Sensorchip 31 erreichen kann und dort zu entsprechenden Messsignalen führt.

Die Messkammer 25 trägt an ihrem unteren Ende nach unten vorstehende, sich über einen Teil des Umfanges erstreckende Stützvorsprünge 33 und an ihrem Deckel 27 radial abstehende Zentrierstege 34, so dass die Messkammer 25 beim Einsetzen in das Unterteil 17 der Flüssigkeitskammer 16 in einer genau definierten Position angeordnet wird, dabei stützen sich die Stützvorsprünge 33 am Boden 18 des Unterteils 17 ab, die radialen Zentrierstege 34 entweder an der Wand 19 des Unterteils 17 oder nach dem Verschließen der Flüssigkeitskammer am Ring 22. In jedem Fall wird durch diese Anordnung erreicht, dass die Messkammer 25 allseits von der Hirnflüssigkeit umströmt werden kann, d.h. die Messkammer 25 hat von dem Boden 18, von den Wänden 19 und auch von dem deckelförmigen Gehäuseteil 21 überall einen Abstand, so dass durch diese Zwischenräume die Hirnflüssigkeit ungehindert strömen kann.

Dies ist insbesondere dann von Bedeutung, wenn mittels der flexiblen Membran 23 ein Rückspül- oder Druckimpuls auf die Hirnflüssigkeit übertragen werden soll, es ist aber auch von Bedeutung, dass durch die freie Durchströmbarkeit des Innenraums 24 keine Verfälschungen bei der Druckmessung auftreten.

Die Messkammer 25 kann in die Flüssigkeitskammer 16 eingesetzt werden, nachdem diese implantiert ist, und es ist auch ohne Weiteres möglich, die Messkammer auszutauschen, wenn sich Fehlfunktionen der Messkammer zeigen sollten oder wenn andere Messwerte von Interesse sein sollten.

## Patentansprüche

1. Messeinrichtung (1) für physiologische Parameter mit einer Hirnflüssigkeit aufnehmenden, implantierbaren Flüssigkeitskammer (16), mit einer mit der Hirnflüssigkeit in der Flüssigkeitskammer (16) über eine Membran (30) in Verbindung stehenden Sensoreinrichtung (31, 32), die einen Sensor für einen oder mehrere physiologische Parameter, elektronische Bauelemente und eine Telemetrieeinrichtung zur drahtlosen Übertragung von Signalen des Sensors umfasst, wobei die Flüssigkeitskammer (16) mindestens zwei Gehäuseteile (17, 21) aufweist, die unter Ausbildung eines geschlossenen Innenraums (24) dichtend zusammenfügbar sind und die im nicht zusammengefügten Zustand einen Zugang zu dem Innenraum (24) ermöglichen, und wobei die Sensoreinrichtung (31, 32) in einer allseits geschlossenen Messkammer (25) angeordnet ist, **dadurch gekennzeichnet, dass** die Messkammer (25) als gegenüber der Flüssigkeitskammer (16) selbständig handhabbares Bauteil ausgebildet und in einer definierten Position im Innenraum (24) der Flüssigkeitskammer (16) einsetzbar ist.

2. Messeinrichtung nach Anspruch 1, **dadurch gekennzeichnet, dass** der Innenraum (24) über mindestens einen Zufluss (13) mit einer Sonde (5) in Verbindung steht, über die Hirnflüssigkeit von einer Messstelle des Hirns (6) in den Innenraum (24) einströmen kann.

3. Messeinrichtung nach Anspruch 1 oder 2, **dadurch gekennzeichnet, dass** der Innenraum (24) mindestens einen Abfluss (20) für Hirnflüssigkeit aufweist.

4. Messeinrichtung nach einem der voranstehenden Ansprüche, **dadurch gekennzeichnet, dass** die Flüssigkeitskammer (16) ein erstes Gehäuseteil (17) mit einem Boden (18) und daran anschließenden Seitenwänden (19) und ein zweites Gehäuseteil (21) aufweist, welches als dichtend auf das erste Gehäuseteil (17) aufsetzbarer Deckel ausgebildet ist.

5. Messeinrichtung nach einem der voranstehenden Ansprüche, **dadurch gekennzeichnet, dass** ein Bereich der Wand der Flüssigkeitskammer (16) flexibel ausgebildet ist, während die übrigen Bereiche der Wand starr ausgebildet sind.

6. Messeinrichtung nach Anspruch 5, **dadurch gekennzeichnet, dass** der flexible Wandbereich durch eine flexible Membran (23) gebildet wird.

7. Messeinrichtung nach Anspruch 6, **dadurch gekennzeichnet, dass** die flexible Membran (23) aus Silikon besteht.

8. Messeinrichtung nach Anspruch 5 oder 6, **dadurch gekennzeichnet, dass** die flexible Membran (23) in einen Dichtring (22) eingesetzt ist, der zusammen mit der Membran (23) ein Gehäuseteil (21) ausbildet, welches mit einem zweiten Gehäuseteil (17) zur Bildung der Flüssigkeitskammer (16) zusammenfügbar ist.

9. Messeinrichtung nach einem der voranstehenden Ansprüche, **dadurch gekennzeichnet, dass** die Messkammer (25) derart in die Flüssigkeitskammer (16) eingesetzt ist, dass sie allseits von Hirnflüssigkeit umgeben ist.

10. Messeinrichtung nach Anspruch 9, **dadurch gekennzeichnet, dass** zwischen der Flüssigkeitskammer (16) und der Messkammer (25) zur Festlegung der Messkammer (25) in der Flüssigkeitskammer (16) Fixiervorsprünge (33, 34) angeordnet sind, die Teil der Flüssigkeitskammer (16) und/oder der Messkammer (25) sind.

11. Messeinrichtung nach einem der voranstehenden Ansprüche, **dadurch gekennzeichnet, dass** die Messkammer (25) aus Metall besteht.

12. Messeinrichtung nach einem der voranstehenden Ansprüche, **dadurch gekennzeichnet, dass** die Messkammer (25) starre Wände aufweist.

13. Messeinrichtung nach einem der voranstehenden Ansprüche, **dadurch gekennzeichnet, dass** die Flüssigkeitskammer (16) aus einem sterilisierbaren Kunststoff besteht.

14. Messeinrichtung nach Anspruch 13, **dadurch gekennzeichnet, dass** die Flüssigkeitskammer (16) aus Polyetheretherketon besteht.

## Claims

1. Measuring device (1) for physiological parameters with an implantable fluid chamber (16) accommodating brain fluid, with a sensor device (31, 32) communicating with the brain fluid in the fluid chamber (16) via a membrane (30) and comprising a sensor for one or more physiological parameters, electronic components and a telemetry device for wireless transmission of signals of the sensor, wherein the fluid chamber (16) comprises at least two housing parts (17, 21) which are adapted to be fitted together in a sealed manner, thereby forming a closed interior (24), and which allow access to the interior (24) when not fitted together, and wherein the sensor device (31, 32) is arranged in a measurement chamber (25) closed on all sides, **characterized in that** the measurement chamber (25) is configured as a component that is adapted for independent handling in relation to the fluid chamber (16) and is insertable in a defined position in the interior (24) of the fluid chamber (16).

2. Measuring device in accordance with claim 1, **characterized in that** the interior (24) is connected via at least one inlet (13) to a probe (5) via which brain fluid can flow from a measuring point of the brain (6) into the interior (24).

3. Measuring device in accordance with claim 1 or 2, **characterized in that** the interior (24) has at least one outlet (20) for brain fluid.

4. Measuring device in accordance with any one of the preceding claims, **characterized in that** the fluid chamber (16) comprises a first housing part (17) with a bottom (18) and adjoining side walls (19) and a second housing part (21) which is configured as a cover that is adapted to be placed in a sealed manner on the first housing part (17).

5. Measuring device in accordance with any one of the preceding claims, **characterized in that** one region of the wall of the fluid chamber (16) is of flexible construction whereas the remaining regions of the wall are of rigid construction.

6. Measuring device in accordance with claim 5, **characterized in that** the flexible wall region is formed by a flexible membrane (23).

7. Measuring device in accordance with claim 6, **characterized in that** the flexible membrane (23) consists of silicone.

8. Measuring device in accordance with claim 5 or 6, **characterized in that** the flexible membrane (23) is inserted into a sealing ring (22) which together with the membrane (23) forms a housing part (21) which is adapted to be fitted together with a second housing part (17) in order to form the fluid chamber (16).

9. Measuring device in accordance with any one of the preceding claims, **characterized in that** the measurement chamber (25) is inserted into the fluid chamber (16) in such a way that it is surrounded on all sides by brain fluid.

10. Measuring device in accordance with claim 9, **characterized in that** fixing projections (33, 34) which are part of the fluid chamber (16) and/or the measurement chamber (25) are arranged between the fluid chamber (16) and the measurement chamber (25) for securing the measurement chamber (25) in the fluid chamber (16).

11. Measuring device in accordance with any one of the preceding claims, **characterized in that** the measurement chamber (25) consists of metal.

12. Measuring device in accordance with any one of the preceding claims, **characterized in that** the measurement chamber (25) has rigid walls.

13. Measuring device in accordance with any one of the preceding claims, **characterized in that** the fluid chamber (16) consists of a sterilizable plastic material.

14. Measuring device in accordance with claim 13, **characterized in that** the fluid chamber (16) consists of polyetheretherketone.

## Revendications

1. Dispositif de mesure (1) pour des paramètres physiologiques, comprenant une chambre de liquide (16) implantable recevant un liquide céphalo-rachidien, comprenant également un dispositif de capteur (31, 32) qui est en liaison avec le liquide céphalo-rachidien dans la chambre de liquide (16) par l'intermédiaire d'une membrane (30), et comporte un capteur pour un ou plusieurs paramètres physiologiques, des composants électroniques et un système de télémétrie pour la transmission sans fil de signaux du capteur, la chambre de liquide (16) présentant au moins deux parties de carter (17, 21) qui peuvent être assemblées de manière étanche en formant une enceinte intérieure (24) fermée, et qui dans l'état non assemblé, permettent un accès à l'enceinte intérieure (24), et le dispositif de capteur (31, 32) étant agencé dans une chambre de mesure (25) fermée de tous les côtés,
**caractérisé en ce que** la chambre de mesure (25) est réalisée sous la forme d'une pièce pouvant être manipulée de manière indépendante et autonome par rapport à la chambre de liquide (16), et peut être insérée dans une position définie dans l'enceinte intérieure (24) de la chambre de liquide (16).

2. Dispositif de mesure selon la revendication 1, **caractérisé en ce que** l'enceinte intérieure (24) est en liaison avec une sonde (5) par l'intermédiaire d'au moins une entrée d'écoulement (13), par laquelle du liquide céphalo-rachidien peut s'écouler, à partir d'une zone de mesure de la cavité encéphalique (6), dans l'enceinte intérieure (24).

3. Dispositif de mesure selon la revendication 1 ou la revendication 2, **caractérisé en ce que** l'enceinte intérieure (24) présente au moins une sortie d'écoulement (20) pour le liquide céphalo-rachidien.

4. Dispositif de mesure selon l'une des revendications précédentes, **caractérisé en ce que** la chambre de liquide (16) présente une première partie de carter (17) avec un fond (18) et des parois latérales (19) qui s'y raccordent, et une deuxième partie de carter (21), qui est réalisée sous la forme d'un couvercle pouvant être rapporté de manière étanche sur la première partie de carter (17).

5. Dispositif de mesure selon l'une des revendications précédentes, **caractérisé en ce qu'**une zone de la paroi de la chambre de liquide (16) est de configuration flexible, tandis que les zones restantes de la paroi sont de configuration rigide.

6. Dispositif de mesure selon la revendication 5, **caractérisé en ce que** la zone de paroi flexible est formée par une membrane flexible (23).

7. Dispositif de mesure selon la revendication 6, **caractérisé en ce que** la membrane flexible (23) est réalisée en silicone.

8. Dispositif de mesure selon la revendication 5 ou la revendication 6, **caractérisé en ce que** la membrane flexible (23) est insérée dans une bague d'étanchéité (22) qui forme, en commun avec la membrane (23), une partie de carter (21) pouvant être assemblée avec une deuxième partie de carter (17) pour former la chambre de liquide (16).

9. Dispositif de mesure selon l'une des revendications précédentes, **caractérisé en ce que** la chambre de mesure (25) est insérée dans la chambre de liquide (16) de manière à être entourée de tous les côtés par du liquide céphalo-rachidien.

10. Dispositif de mesure selon la revendication 9, **caractérisé en ce que** pour immobiliser la chambre de mesure (25) dans la chambre de liquide (16), entre la chambre de liquide (16) et la chambre de mesure (25) sont agencées des protubérances de fixation (33, 34), qui font partie de la chambre de liquide (16) et/ou de la chambre de mesure (25).

11. Dispositif de mesure selon l'une des revendications précédentes, **caractérisé en ce que** la chambre de mesure (25) est réalisée en métal.

12. Dispositif de mesure selon l'une des revendications précédentes, **caractérisé en ce que** la chambre de mesure (25) présente des parois rigides.

13. Dispositif de mesure selon l'une des revendications précédentes, **caractérisé en ce que** la chambre de liquide (16) est réalisée en une matière plastique pouvant être stérilisée.

14. Dispositif de mesure selon la revendication 13, **caractérisé en ce que** la chambre de liquide (16) est réalisée en polyétheréthercétone.
